(19) Europäisches Patentamt
European Patent Office
Office européen des brevets

(11) EP 1 795 231 A2

(12) **DEMANDE DE BREVET EUROPEEN**

(43) Date de publication:
**13.06.2007 Bulletin 2007/24**

(21) Numéro de dépôt: **06301225.6**

(22) Date de dépôt: **07.12.2006**

(51) Int Cl.:
***A61Q 1/04*** *(2006.01)* ***A61K 8/37*** *(2006.01)*
***A61K 8/31*** *(2006.01)* ***A61K 8/58*** *(2006.01)*
***A61K 8/81*** *(2006.01)*

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **08.12.2005 FR 0553798**

(71) Demandeur: **L'ORÉAL**
**75008 Paris (FR)**

(72) Inventeurs:
- **Shimizu, Momoko**
  **Tokyo 150-0001 (JP)**
- **Tokunaga, Emiko**
  **Kanagawa 213-0006 (JP)**

(74) Mandataire: **Le Coupanec, Pascale A.M.P.**
**Nony & Associés,**
**3 rue de Penthièvre**
**75008 Paris (FR)**

(54) **Composition cosmétique comprenant un ester d'acide dimerdilinoléique et de polyol(s), une huile volatile et un agent filmogène**

(57) La présente invention concerne une composition cosmétique anhydre de soin et/ou de maquillage de la peau et/ou des lèvres comprenant au moins un ester d'acide dimerdilinoléique et de polyol(s) ou un de ses esters, dont la viscosité, mesurée à environ 25 °C, est supérieure ou égale à environ 20 000 mPa.s, au moins un agent filmogène et au moins une huile volatile.

EP 1 795 231 A2

## Description

La présente invention a pour objet des compositions cosmétiques de soin et/ou de maquillage de la peau et/ou des lèvres comprenant un ester d'acide dimerdilinoléique et de polyol(s) ou un de ses esters, un agent filmogène, une huile volatile.

Les compositions selon l'invention peuvent présenter une brillance et/ou une tenue de la couleur améliorées.

La présente invention concerne également un procédé de maquillage du visage et/ou du corps humain mettant en oeuvre une composition selon l'invention.

Une composition selon l'invention peut, en particulier, être un produit de maquillage et/ou de soin destiné à être appliqué sur le corps, par exemple le visage et/ou les lèvres, et être notamment un rouge à lèvres, un baume à lèvres, un crayon à lèvres, un fond de teint liquide ou solide, notamment coulé en stick ou en coupelle, un produit anti-cerne, un produit de coloration de la peau, un produit de maquillage des yeux, comme un eye-liner, en particulier sous forme de crayon, un mascara notamment sous la forme de pain ou encore de fard à paupières, ou un brillant à lèvres.

De nombreuses compositions cosmétiques existent pour lesquelles des propriétés de brillance et/ou d'effet coloré du film déposé, après application sur la peau et/ou les lèvres, sont recherchées. Ces propriétés participent généralement à l'effet esthétique recherché.

Par ailleurs, il est également requis que cet effet esthétique se maintienne au cours du temps, notamment face aux différents facteurs extérieurs susceptibles d'en affecter les caractères de brillance et/ou de couleur.

Afin de conférer aux compositions cosmétiques de bonnes propriétés de brillance et notamment une bonne tenue de la brillance, il a été proposé d'utiliser des huiles dites "brillantes", telles que des polymères huileux comme des polybutènes de viscosité élevée, des esters d'acides ou d'alcools gras dont le nombre de carbone est élevé (typiquement supérieur à 16), ou encore certaines huiles végétales.

Toutefois, ces composés peuvent dans certaines circonstances être collants à l'application et/ou au cours du temps, ce qui peut entraîner d'importantes sensations désagréables et un sentiment d'inconfort pour l'utilisateur.

De manière surprenante, les inventeurs ont pu observer que la mise en oeuvre de certains esters d'acide dimerdilinoléique et de polyol(s) ou un de ses esters en combinaison avec un agent filmogène, par exemple une résine MQ ou un polymère PSPA, pour préparer des compositions cosmétiques permettait de conférer à ces dernières une tenue de la couleur améliorée sans affecter l'ensemble de leur propriétés esthétiques, notamment l'effet de brillance et la tenue de la brillance, tout en procurant une sensation de confort à l'application.

Par exemple, une composition cosmétique selon l'invention peut posséder la propriété avantageuse de ne pas provoquer de sensation collante après son application.

Ainsi, la présente invention a pour objet, selon un de ses aspects, une composition cosmétique anhydre de soin et/ou de maquillage de la peau et/ou des lèvres comprenant au moins un ester d'acide dimerdilinoléique et de polyol(s) ou un de ses esters, dont la viscosité, mesurée à environ 25 °C, est supérieure ou égale à environ 20 000 mPa.s, au moins un agent filmogène et au moins une huile volatile.

Au sens de l'invention, on entend par "composition anhydre", une composition contenant moins de 5 % en poids, en particulier moins de 3 % en poids, en particulier moins de 2 % en poids, et plus particulièrement moins de 1 % en poids d'eau par rapport au poids total de la composition.

Selon un autre de ses aspects, la présente invention a pour objet une composition cosmétique de soin et/ou de maquillage de la peau et/ou des lèvres comprenant au moins un ester de formule générale (I) suivante :

$$R_3\text{-OCO-}R_1(\text{-COO-}R_2\text{-OCO-}R_1)_n\text{-COO-}R_3 \qquad \text{(I)}$$

dans laquelle :

- $COR_1CO$ représente un résidu dimerdilinoléate,
- $OR_2O$ représente un résidu de dimère d'alcool gras,
- $OR_3$ représente un résidu de monoalcool hydrocarboné, et
- n est un entier variant de 1 à 15,

au moins un agent filmogène et au moins une huile volatile.

Selon un autre de ses aspects, la présente invention a pour objet une composition cosmétique de soin et/ou de maquillage de la peau et/ou des lèvres comprenant au moins un ester de formule générale (II) suivante :

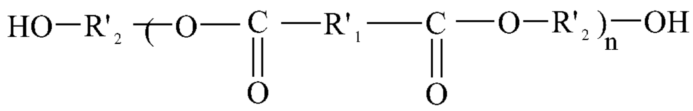

(II)

dans laquelle :

- n est un entier variant de 1 à 15,
- OCOR'1CO représente un résidu dimerdilinoléate,
- OR'2O représente un résidu diglycéryle de formule générale (III) suivante :

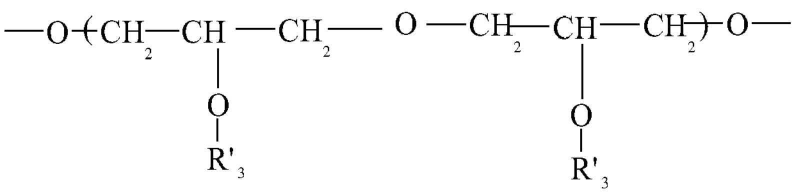

(III)

dans laquelle :

- $R'_3$ représente H ou $OR'_3$ représente un résidu d'acide gras,

au moins un agent filmogène et au moins une huile volatile.

Selon un autre de ses aspects, la présente invention a pour objet l'utilisation d'au moins un ester d'acide dimerdilinoléique et de polyol(s) ou d'un de ses esters conformes à l'invention en association avec au moins une huile volatile et au moins un agent filmogène pour la préparation d'une composition cosmétique de soin et/ou de maquillage de la peau et/ou des lèvres présentant une tenue moyenne de la couleur améliorée.

Selon encore un autre de ses aspects, la présente invention a pour objet un procédé de maquillage et/ou de soin de la peau et/ou des lèvres comprenant au moins une étape consistant à appliquer sur au moins une partie d'un support une composition conforme à l'invention.

Selon un de ses avantages, la présente invention permet de fournir des compositions cosmétiques dont la tenue de la brillance et/ou la brillance n'est pas affectée, voire est améliorée, tout en présentant une tenue de la couleur améliorée.

Selon un autre de ses avantages, la présente invention permet de fournir des compositions dont la tenue de la couleur est améliorée au cours du temps, notamment face aux différentes agressions extérieures, telles que la prise d'un repas ou le contact avec un tissu.

Selon un autre de ses avantages, la présente invention permet de fournir des compositions cosmétiques n'entraînant pas de sensations collantes ou d'inconfort à l'application.

## ESTER D'ACIDE DIMERDILINOLEIQUE ET DE POLYOL(S)

Dans l'expression "ester d'acide dimerdilinoléique et de polyol(s) ou un de ses esters", on entend désigner par "ou un de ses esters", un des dérivés de ces esters acide dimerdilinoléique et de polyol(s) obtenu soit par réaction de fonction(s) alcool du polyol, non engagée(s) dans des liaisons de type ester avec des fonctions acide de l'acide dilinoléique, avec une ou plusieurs fonctions carboxylique de molécules d'acides autre que l'acide dilinoléique ou encore par réaction de fonction(s) acide du dimère dilinoléique, non engagée(s) dans des liaisons de type ester avec des fonctions alcool du polyol, avec des fonctions alcool de molécules d'alcools distinctes du polyol.

### Acide dimerdilinoléique

L'acide dimerdilinoléique convenant à la mise en oeuvre de la présente invention peut être obtenu par réaction de polymérisation, notamment de dimérisation intermoléculaire d'un acide linoléique.

La stabilité du composé vis-à-vis de l'oxydation peut être améliorée par hydrogénation des doubles liaisons restantes après la réaction de dimérisation.

L'acide dimerdilinoléique peut être également obtenu par dimérisation de la forme hydrogénée de l'acide linoléique.

La forme hydrogénée de l'acide ou du diacide peut être partielle ou totale, et par exemple correspondre à la forme saturée, plus stable à l'oxydation.

Comme indiqué précédemment, les fonctions carboxylique du résidu d'acide dimerdilinoléique non engagées dans la liaison ester avec le ou les résidus polyols peuvent être engagées dans d'autres liaisons ester avec d'autres fonctions alcool de molécules d'alcool distinctes du ou des polyol(s).

Ces molécules ou résidus d'alcools peuvent être des monoalcools ou des polyols.

A titre d'exemple de résidu d'alcool convenant à la mise en oeuvre de l'invention, on peut mentionner les composés hydrocarbonés comprenant une fonction hydroxyle et comprenant de 4 à 40 atomes de carbone, en particulier de 6 à 36 atomes de carbone, en particulier de 8 à 32 atomes de carbone, en particulier de 16 à 28 atomes de carbone, et plus particulièrement de 18 à 24 atomes de carbone.

A titre d'exemple de monoalcool convenant à l'invention, on peut citer, de manière non limitative, le butanol, le pentanol, le propanol, l'hexanol, l'heptanol, l'octanol, le décanol, le dodécanol, l'hexadécanol, l'octadécanol, l'eicosadécanol, le phytostérol, l'isostéarol, le stéarol, le cétol, le béhénol, etc.

<u>Polyols</u>

Par le terme "polyol", on entend couvrir tout composé hydrocarboné comprenant au moins deux fonctions hydroxyle et comprenant de 4 à 40 atomes de carbone, en particulier de 6 à 36 atomes de carbone, en particulier de 8 à 32 atomes de carbone, en particulier de 16 à 28 atomes de carbone, et plus particulièrement de 18 à 24 atomes de carbone.

Les chaînes hydrocarbonées peuvent, le cas échéant, être interrompues par la présence d'au moins un hétéroatome, et notamment un atome d'oxygène.

Un polyol ou un ester de polyol convenant à la mise en oeuvre de la présente invention peut comprendre, par exemple, de 2 à 12 fonctions hydroxyle, en particulier de 2 à 8 fonctions hydroxyle, et plus particulièrement de 4 à 6 fonctions hydroxyle.

Le cas échéant, les fonctions hydroxyle, autres que celles déjà engagées dans une liaison ester avec l'acide dimerdilinoléique peuvent être également engagées, toutes ou en partie dans d'autres liaisons ester après réaction avec des molécules d'acides autres que l'acide dimerdilinoléique.

Le polyol ou un de ses esters convenant à la mise en oeuvre de la présente invention peut être, notamment, choisi parmi les alcools linéaires, ramifiés, cycliques ou polycycliques, saturés ou insaturés.

Ainsi, le polyol peut être choisi par exemple parmi un diol, un triol, un tétraol, ou un pentaol, ou un de leurs esters.

Le polyol peut être un diol, ou un de ses esters, notamment choisi parmi un dimère d'alcool gras, un mono- ou poly-glycérol, un mono- ou poly-alkylène en $C_{2-4}$ glycol, le 1,4 butanediol, et le pentaérythritol.

A titre d'exemple de diol pouvant également convenir à la mise en oeuvre de l'invention, on peut citer, de manière non exhaustive, le butadiol, le pentadiol, le propanediol, l'hexanediol, l'hexylèneglycol, l'heptanediol, l'octanediol, le nonanediol, le décanediol, l'un-décanediol, le dodécanediol, le tridécanediol, le tétradécanediol, le pentadécanediol, l'hexadécanediol, le nonadécanediol, l'octadecènediol, le cyclohexanediol, le diglycérol, l'érythritol, le pentaérythritol, le xylitol, le sorbitol, l'éthylèneglycol, le xylèneglycol et leurs isomères.

Un dimère d'alcool gras peut également être le produit d'hydrogénation, par exemple catalytique, d'un dimère d'acide gras, lui-même obtenu par dimérisation d'un acide gras insaturé notamment en $C_8$ à $C_{34}$, notamment en $C_{12}$ à $C_{22}$, en particulier en $C_{16}$ à $C_{20}$, et plus particulièrement en $C_{18.}$

Un dimère d'alcool gras peut varier de $C_{16}$ à $C_{68}$, en particulier de $C_{24}$ à $C_{44}$, en particulier de $C_{32}$ à $C_{40}$ et plus particulièrement être en $C_{36}$.

Selon un mode de réalisation particulier, le dimère d'alcool gras peut être un dimère diol dérivant de l'hydrogénation du diacide dilinoléique. Il est généralement sous une forme saturée.

Un dimère d'alcool gras peut, par exemple, être un dimère de dilinoléol.

A titre d'exemple de diol susceptible de convenir à la mise en oeuvre de l'invention on peut notamment citer le diglycérol.

Ce composé est un dimère de glycérol résultant de la condensation de deux molécules de glycérol avec perte d'une molécule d'eau.

On entend par "diglycérol", l'ensemble des isomères susceptibles de résulter d'une telle condensation, comme par exemple les isomères linéaires, les isomères ramifiés et le cas échéant, les isomères cycliques résultant d'une déshydratation intra-moléculaire d'une molécule de diglycérol.

Le diglycérol peut être obtenu par tout procédé connu de l'homme de l'art et notamment ceux décrits dans le brevet EP 0 750 848.

A titre d'exemple de molécules d'acides susceptibles d'interagir avec une ou plusieurs fonctions hydroxyle du polyol, non engagée(s) dans la liaison ester avec l'acide dimerdilinoléique on peut mentionner, de manière non limitative, les molécules dérivées de l'acide isostéarique, l'acide béhénique, l'acide phytostérique, l'acide stéarique ou l'acide

cétylique.

Un ester convenant à la mise en oeuvre de la présente invention peut être obtenu par réaction d'un polyol ou un de ses esters avec un acide dimerdilinoléique, selon un rapport molaire d'environ 1,0:0,2-1,0.

Un ester susceptible de convenir à la mise en oeuvre de la présente invention peut, notamment, être obtenu par réaction d'un acide dimerdilinoléique avec un dilinoléol, et le cas échéant, au moins un monoalcool additionnel, notamment choisi parmi le béhénol, l'isostéarol, le phytostérol, le stéarol, le cétol et leurs mélanges.

Ainsi, un ester mis en oeuvre dans le cadre de la présente invention peut être utilisé sous la forme d'un mélange de différents esters par exemple.

Un ester convenant à l'invention peut par exemple être obtenu par réaction d'un glycérol, d'un acide isostéarique et d'un acide dimerdilinoléique, notamment, selon un rapport molaire de 1,0:0,2-1,0:0,5-0,9.

A titre d'exemple d'ester d'acide dimerdilinoléique et de polyol(s) ou un de ses esters convenant à l'invention, on peut citer les esters décrits dans les demandes JP 2004-256515 et JP 2005-179377.

Un ester d'acide dilinoléique et de polyol(s) ou un de ses esters convenant à la mise en oeuvre de la présente invention peut présenter un poids moléculaire variant d'environ 2 000 à environ 25 000 g/mol, en particulier d'environ 4 000 à environ 20 000 g/mol, en particulier d'environ 7 000 à environ 15 000 g/mol, et plus particulièrement d'environ 8 000 à environ 10 000 g/mol.

Selon un mode de réalisation, un ester conforme à l'invention peut comprendre un enchaînement alterné de résidu(s) dimerdilinoléate(s) et de résidu(s) apparenté(s) au(x)dit(s) polyol(s), et notamment au(x)dit(s) diol(s), le(s)dit (s) polyol(s) ou diol(s) étant, par exemple, tel(s) que défini(s) précédemment.

Ainsi, dans une telle configuration, chacune des deux extrémités dudit enchaînement peut porter respectivement un motif OR' et OR", avec R' et R" représentant, indépendamment l'un de l'autre, un atome d'hydrogène ou OR' et OR" représentant, indépendamment l'un de l'autre, un résidu d'un monoalcool hydrocarboné en $C_2$ à $C_{36}$, notamment en $C_8$ à $C_{24}$, en particulier en $C_{12}$ à $C_{20}$, et plus particulièrement en $C_{16}$ à $C_{18}$.

Selon un mode de réalisation, R' et R" peuvent représenter tous les deux un atome d'hydrogène.

Selon un mode de réalisation, OR' et OR" peuvent représenter tous les deux un résidu de monoalcool hydrocarboné, identique ou différent.

A titre d'exemple de résidus de monoalcool hydrocarboné OR' et OR" pouvant convenir à l'invention, on peut mentionner les résidus d'alcools gras.

Selon un mode de réalisation, un ester d'acide dimerdilinoléique et de polyol(s) ou un de ses esters pouvant convenir à la mise en oeuvre de la présente invention peut être de formule générale (I) suivante :

$$R_3\text{-}OCO\text{-}R_1(\text{-}COO\text{-}R_2\text{-}OCO\text{-}R_1)_n\text{-}COO\text{-}R_3 \quad (I)$$

dans laquelle :

- $COR_1CO$ représente un résidu dimerdilinoléate,
- $OR_2O$ représente un résidu de dimère d'alcool gras pouvant varier de $C_{16}$ à $C_{68}$, en particulier de $C_{24}$ à $C_{44}$, en particulier de $C_{32}$ à $C_{40}$ et plus particulièrement être en $C_{36}$,
- $OR_3$ représente un résidu de monoalcool pouvant varier de $C_4$ à $C_{40}$, en particulier de $C_6$ à $C_{36}$, en particulier de $C_8$ à $C_{32}$, en particulier de $C_{16}$ à $C_{28}$, et plus particulièrement de $C_{18}$ à $C_{24}$, et
- n est un entier variant de 1 à 15, en particulier de 2 à 10 et plus particulièrement de 5 à 7.

Selon une variante de réalisation, $OR_2O$ peut représenter un résidu dimerdilinoléyle.

Par ailleurs $OR_3$ peut représenter un résidu de monoalcool hydrocarboné choisi, par exemple, parmi les résidus béhényle, isostéaryle, phytostéryle, et leurs mélanges.

Selon une autre variante de réalisation, l'ester d'acide dimerdilinoléique et de polyol(s) ou un de ses esters pouvant convenir à la mise en oeuvre de l'invention peut être, notamment, de formule générale (II) suivante :

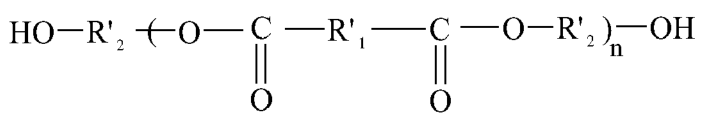

(II)

dans laquelle :

- n est un entier variant de 1 à 15, notamment de 2 à 10 et en particulier de 5 à 7,
- OCR'1CO représente un résidu dimerdilinoléate,

- OR'2O représente un résidu diglycéryle de formule générale (III) suivante :

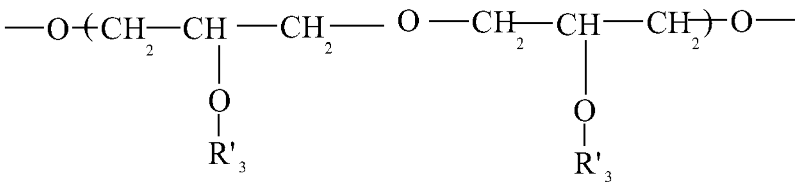

(III)

dans laquelle :

- $R'_3$ représente H ou $OR'_3$ représente un résidu d'acide gras pouvant varier de $C_8$ à $C_{34}$, en particulier de $C_{12}$ à $C_{22}$, en particulier de $C_{16}$ à $C_{32}$ et plus particulièrement étant en $C_{18}$.

Selon une variante de réalisation, le résidu d'acide gras figuré par $OR'_3$ peut être un résidu d'isostéaryle.

La viscosité d'un ester d'acide dimerdilinoléique et de polyol(s) ou un de ses esters selon l'invention peut être mesurée selon tout procédé connu de l'homme de l'art, et notamment selon le procédé conventionnel décrit par la suite.

La viscosité peut être mesurée au moyen d'un viscosimètre cône/plateau ou à plaques parallèles de type ARES (TA-INSTRUMENT) et fonctionnant selon un mode en balayage de cinétique sur une gamme de cisaillement d'environ 1-1000 $s^{-1}$ pour induire une tension d'écoulement d'environ de 1000 Pa.

Le cône/plateau ou les plaques parallèles peuvent être consituées d'un matériau choisi parmi l'acier inoxydable, les résines acryliques ou le sulfure de polyphénylène (résine PPS).

Le diamètre cône/plateau peut être de 25 mm (angle de cône 0,10 radiants).

La mesure est réalisée à environ 25 °C.

Avant toute mesure, la stabilité de l'échantillon est vérifiée par le test de la période de balayage dynamique qui permet de déterminer si l'échantillon est stable par lui-même.

La viscosité de cisaillement est déterminée en utilisant la valeur de ETA dans la région du plateau selon l'écoulement.

La période de balayage dynamique est déterminée à une fréquence de 1,0 Hz sur une période de 600 secondes.

Les mesures à vitesse de balayage constante sont réalisées avec une vitesse variant de 1,0 à 1000 $s^{-1}$, en particulier de 1,0 à 100 $s^{-1}$.

La viscosité d'un ester d'acide dimerdilinoléique et de polyol ou d'un de ses esters convenant à la mise en oeuvre de l'invention peut varier d'environ 20 000 mPa.s à environ 150 000 mPa.s, notamment d'environ 40 000 mPa.s à environ 100 000 mPa.s, et en particulier d'environ 60 000 mPa.s à environ 80 000 mPa.s.

Un ester convenant à l'invention peut notamment être choisi parmi les esters de nomenclature INCI suivante : le copolymère d'isostéarate de polyglyceryle-2/dimerdilinoléate, le bis-béhényl/isostéaryl/phytostéryl dimerdilinoléyle dimerdilinoléate, et leurs mélanges.

De tels composés peuvent être obtenus, par exemple, sous la référence HAILUSCENT ISDA (KOKYU ALCOHOL), et PLANDOOL-G (NIPPON FINE CHEMICAL COMPANY, Ltd.).

Un ester d'acide dimerdilinoléique et de polyol(s) ou un de ses esters convenant à la mise en oeuvre de l'invention peut être présent dans les compositions cosmétiques selon l'invention en une quantité suffisante pour conférer à ces compositions des propriétés cosmétiques améliorées, notamment en terme de tenue de la brillance.

L'ester peut être présent en une teneur variant d'environ 0,1 % à environ 80 % en poids, en particulier d'environ 2 % à environ 70 % en poids et plus particulièrement d'environ 30 % à environ 50 % en poids par rapport au poids total de la composition.

## AGENT FILMOGENE

Une composition cosmétique selon l'invention contient au moins un agent filmogène qui peut être notamment choisi parmi les polymères filmogènes. Certains polymères filmogènes peuvent être gélifiants.

Un agent filmogène peut être, dans une composition selon l'invention, associé ou non avec au moins un auxiliaire de filmification.

Par "polymère", on entend au sens de l'invention un composé ayant au moins 2 motifs de répétition, et de préférence au moins 3 motifs de répétition.

Par "polymère filmogène", on entend désigner un polymère apte à former à lui seul ou en présence d'un agent auxiliaire de filmification, un film macroscopiquement continu sur un support, notamment sur les matières kératiniques, et en particulier un film cohésif et notamment un film dont la cohésion et les propriétés mécaniques sont telles que ledit film peut être isolé dudit support.

Un polymère filmogène peut être présent dans une composition cosmétique selon l'invention en une teneur variant d'environ 0,1 % à environ 60 % en poids, notamment variant d'environ 0,5 % à environ 60 % en poids, en particulier variant d'environ 1 % à environ 50 % en poids, en particulier variant d'environ 1 % à environ 40 % en poids, en particulier variant d'environ 2 % à environ 30 % en poids, et plus particulièrement variant d'environ 5 % à environ 25 % en poids de polymère filmogène par rapport au poids total de la composition.

Introduit en quantité suffisante, en combinaison avec un ester d'acide dimerdilinoléique et de polyol(s) ou un de ses esters tel que défini précédemment et une huile volatile telle que définie ci-après, l'agent filmogène présente notamment l'avantage de pouvoir conférer une amélioration de la tenue de la brillance, voire également de la tenue de la couleur aux compositions cosmétiques selon l'invention.

Dans un mode de réalisation, un polymère convenant à l'invention peut être choisi parmi les polymères filmogènes solubles dans un milieu liquide organique, les polymères filmogènes dispersibles dans un milieu liquide organique, les polymères filmogènes sous forme de dispersions aqueuses de particules de polymères, les polymères filmogènes hydrosolubles, et leurs mélanges.

Ainsi, un polymère convenant à l'invention peut être choisi parmi :

- les polymères filmogènes solubles dans un milieu liquide organique, par exemple les polymères liposolubles, lorsque le milieu liquide organique comprend au moins une huile ;
- les polymères filmogènes dispersibles dans un milieu liquide organique, par exemple les polymères sous la forme de dispersions non aqueuses de particules de polymères, de préférence des dispersions dans les huiles siliconées ou hydrocarbonées ; dans un mode de réalisation, les dispersions non aqueuses de polymère peuvent comprendre des particules de polymères stabilisés sur leur surface par au moins un agent stabilisant ;
- les polymères filmogènes sous forme de dispersions aqueuses de particules de polymères, souvent appelées "latex" ; dans ce cas, la composition peut comprendre une phase aqueuse ;
- les polymères filmogènes hydrosolubles ; dans ce cas, la composition peut comprendre une phase aqueuse,
- et leurs mélanges.

Comme polymères filmogènes également utilisables dans une composition cosmétique selon la présente invention, on peut citer les polymères synthétiques, de type radicalaire ou de type polycondensat, les polymères d'origine naturelle tels que ceux décrits dans la demande FR 04 50540 déposée le 18 mars 2004 et leurs mélanges.

Comme polymère filmogène, on peut également citer plus particulièrement les polymères et copolymères acryliques, les polyuréthannes, les polyesters, les polyamides, les polyurées, les polymères cellulosiques comme la nitrocellulose, les polymères siliconés, notamment les résines de silicones, les polymères acrylique greffés silicone, les polymères et copolymères polyamide, et leurs mélanges.

Ainsi, un polymère filmogène convenant à l'invention peut être choisi parmi les polymères filmogènes décrits dans la demande WO 04/028487.

De même, un polymère filmogène convenant à l'invention peut être un composé siliconé réticulé tel que décrit dans les demandes US 2003/0103918 et US 2003/0049216.

Un polymère filmogène convenant à l'invention peut également être choisi parmi ceux décrits dans la demande de brevet internationale déposée sous le n° PCT/FR03/02849.

Un polymère filmogène convenant à l'invention peut être encore un polymère séquencé éthylénique linéaire filmogène. D'une manière générale, un polymère séquencé peut comprendre au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Un polymère filmogène utilisable dans l'invention peut également être sous la forme d'une dispersion de particules, de préférence solides, d'un polymère éthylénique greffé dans une phase grasse liquide. Une telle dispersion est notamment décrite dans la demande de brevet internationale déposée sous le n° de dépôt PCT/FR03/03709.

Ainsi, un tel polymère éthylénique greffé peut comprendre un squelette éthylénique insoluble dans la phase grasse liquide, et des chaînes latérales liées de manière covalente audit squelette et solubles dans ledit milieu de dispersion. Avantageusement, le polymère filmogène peut être un copolymère de nature acrylique et peut plus particulièrement comprendre un ou plusieurs monomères choisis parmi l'acrylate d'éthyle-2-hexyle, l'acrylate d'isobutyle, l'acrylate de méthyle, et/ou leurs copolymères.

Les copolymères peuvent être avantageusement solubles ou dispersibles dans les milieux cosmétiques et en particulier dans un milieu aqueux, hydroorganique ou organique. Il peut ainsi être sous la forme d'une dispersion aqueuse de particules ou sous la forme d'une dispersion huileuse de particules.

Selon un mode réalisation, un polymère filmogène convenant à l'invention peut être figuré par au moins un copolymère de type copolymère bloc acrylique ayant au moins une séquence (ou bloc) à conformation limitée et une séquence (ou bloc) à conformation variable. Ces deux séquences sont incompatibles l'une avec l'autre et sont caractérisées par une température de transition vitreuse (Tg) différente.

Ces première et deuxième séquences peuvent être reliées entre elles par un segment intermédiaire comprenant au moins un monomère constitutif de la première séquence et au moins un monomère constitutif de la deuxième séquence.

De tels copolymères sont encore désignés sous le terme "polymère séquencé".

Chaque séquence ou bloc du polymère séquencé est issue d'un type de monomère ou de plusieurs types de monomères différents.

Cela signifie que chaque séquence peut être constituée d'un homopolymère ou d'un copolymère, ce copolymère constituant la séquence pouvant être à son tour statistique ou alterné.

Le segment intermédiaire éventuel comprenant au moins un monomère constitutif de la première séquence et au moins un monomère constitutif de la deuxième séquence du polymère est généralement lui même un polymère statistique.

Le polymère séquencé peut être obtenu par polymérisation radicalaire en solution selon le procédé décrit dans la demande de brevet EP 1 411 069.

Comme indiqué ci-dessus, la séquence à conformation variable et la séquence à conformation limitée ont des températures de transition vitreuse différente.

L'écart entre les températures de transition vitreuse des séquences à conformation limitée et variable est généralement supérieur à 20 °C, de préférence supérieur à 30 °C, et mieux supérieur à 40 °C.

Comme précisé précédemment la séquence ou bloc à conformation variable possède une température de transition vitreuse inférieure à 30 °C, notamment comprise entre -100 °C et +25 °C, par exemple entre -90 °C et +20 °C et mieux comprise entre -80 °C et 0 °C.

Les monomères particulièrement préférés pour la séquence à conformation variable sont les acrylates d'alkyles dont la chaîne alkyle comprend de 1 à 10, voire de 1 à 4 atomes de carbone, tels que l'acrylate de méthyle, l'acrylate d'isobutyle, l'acrylate d'éthyl-2-hexyle et leurs copolymères.

La proportion de la séquence à conformation variable dont la Tg peut être généralement inférieure à 30 °C, voire inférieure à 20 °C, varie de 5 à 75 %, de préférence de 15 à 50 %, et mieux de 25 à 45 % en poids par rapport au poids du polymère séquencé.

La séquence ou bloc ayant une conformation limitée a avantageusement une Tg supérieure ou égale à 40 °C, par exemple une Tg allant de 40 à 120 °C, de préférence supérieure à 50 °C et mieux supérieure à 60 °C.

Les monomères particulièrement préférés pour la séquence à conformation limitée sont le méthacrylate de méthyle, le méthacrylate d'isobutyle, le (méth)acrylate d'isobornyle, le méthacrylate de trifluoroéthyle, le styrène, l'acide (méth)acrylique et leurs copolymères.

La proportion de la séquence à conformation limitée de Tg peut être avantageusement supérieure ou égale à 40 °C varie de 20 à 90 %, mieux de 30 à 80 % et encore mieux de 50 à 70 % en poids par rapport au poids du polymère séquencé.

A titre illustratif et non limitatif des copolymères séquencés convenant à l'invention, on peut plus particulièrement citer les variantes de copolymères suivantes :

- une séquence à conformation limitée de Tg supérieure ou égale à 40 °C, par exemple ayant une Tg allant de 70 à 110 °C, qui est un copolymère méthacrylate de méthyle / acide acrylique, et
- une séquence à conformation variable de Tg inférieure ou égale à 20 °C, par exemple allant de 0 à 20 °C, qui est un homopolymère d'acrylate de méthyle.

Selon une seconde variante, le copolymère selon l'invention peut comprendre :

- une séquence à conformation limitée de Tg supérieure ou égale à 40 °C, par exemple allant de 70 à 100 °C, qui est un copolymère méthacrylate de méthyle/acide acrylique/méthacrylate de trifluoroéthyle, et
- une séquence à conformation variable de Tg inférieure ou égale à 20 °C, par exemple allant de 0 à 20 °C, qui est un homopolymère d'acrylate de méthyle.

Selon une troisième variante, le copolymère selon l'invention, peut comprendre

- une séquence à conformation limitée Tg supérieure ou égale à 40 °C, par exemple allant de 85 à 115 °C, qui est un copolymère acrylate d'isobornyle/méthacrylate d'isobutyle, et
- une séquence à conformation variable de Tg inférieure ou égale à 20 °C, par exemple allant de -85 à -55 °C, qui est un homopolymère d'acrylate d'éthyl-2 hexyle.

Selon une quatrième variante, le copolymère selon l'invention peut comprendre

- une séquence à conformation limitée de Tg supérieure ou égale à 40 °C, par exemple allant de 95 à 125 °C, qui est un copolymère acrylate d'isobornyle/méthacrylate d'isobornyle, et
- une séquence à conformation variable de Tg inférieure ou égale à 20 °C, par exemple allant de -35 à -5 °C, qui est un homopolymère d'acrylate d'isobutyle.

Selon une autre variante de l'invention, le polymère filmogène peut être un polymère de type polymère éthylénique greffé.

Par polymère "éthylénique", on entend un polymère obtenu par polymérisation de monomères comprenant une insaturation éthylénique.

Par polymère greffé, on entend un polymère ayant un squelette comprenant au moins une chaîne latérale pendante ou située en bout de chaîne, et de préférence pendante.

Le polymère greffé peut avoir une masse moléculaire moyenne en poids (Mw) variant de 5 000 à 300 000, notamment de 10 000 à 300 000, notamment de 20 000 à 200 000, mieux encore de 25 000 à 150 000 g/mol.

Avantageusement, le polymère éthylénique greffé peut comprendre un squelette éthylénique insoluble dans ladite phase grasse liquide, et des chaînes latérales liées de manière covalente audit squelette et solubles dans la phase grasse liquide.

Le polymère éthylénique greffé peut être un polymère non réticulé. En particulier, le polymère est obtenu par polymérisation de monomères comprenant un seul groupement polymérisable.

Le polymère éthylénique greffé peut être par exemple susceptible d'être obtenu selon le procédé décrit dans le document WO 04/055081 par polymérisation radicalaire dans un milieu organique de polymérisation :

- d'au moins un monomère éthylénique, en particulier d'au moins un monomère acrylique et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 g/mol et la teneur en macromonomère polymérisé représentant de 0,05 à 20 % en poids du polymère.

Selon un mode de réalisation de l'invention, le polymère éthylénique greffé peut être un polymère acrylique greffé, c'est-à-dire dérivant de la polymérisation d'au moins un monomère acrylique.

Parmi les monomères acryliques susceptibles de former le squelette du polymère éthylénique, on peut tout particulièrement citer les (méth)acrylates de méthyle, d'éthyle, de propyle, de butyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle ; le diméthylaminopropylméthacrylamide; l'acide (méth)acrylique ; et leurs sels ; et leurs copolymères.

De préférence, les monomères acryliques peuvent être choisis parmi l'acrylate de méthyle, l'acrylate de méthoxyéthyle, le méthacrylate de méthyle, le méthacrylate de 2-hydroxyéthyle, l'acide (méth)acrylique, le méthacrylate de diméthylaminoéthyle, et leurs mélanges.

En ce qui concerne les macromonomères, ils peuvent comporter à une des extrémités de la chaîne un groupe terminal polymérisable apte à réagir au cours de la polymérisation avec les monomères acryliques et éventuellement les monomères vinyliques additionnels, pour former les chaînes latérales du polymère éthylénique greffé. Ledit groupe terminal polymérisable peut être en particulier un groupe vinyle ou (méth)acrylate (ou (méth)acryloxy), et de préférence un groupe (méth)acrylate.

Les macromonomères peuvent être choisis préférentiellement parmi les macromonomères dont l'homopolymère a une température de transition vitreuse (Tg) inférieure ou égale à 25 °C, notamment allant de -100 °C à 25 °C, de préférence allant de -80 °C à 0 °C.

Les macromonomères peuvent avoir en général une masse moléculaire moyenne en poids supérieure ou égale à 200 g/mol, de préférence supérieure ou égale à 300 g/mol, préférentiellement supérieure ou égale à 500 g/mol, et plus préférentiellement supérieure à 600 g/mol.

De préférence, les macromonomères peuvent avoir une masse moléculaire moyenne en poids (Mw) allant de 200 à 100 000 g/mol, de préférence allant de 500 à 50 000 g/mol, préférentiellement allant de 800 à 20 000 g/mol, plus préférentiellement allant de 800 à 10 000 g/mol.

Ces macromonomères peuvent être des homopolymères et les copolymères (méth)acrylate d'alkyle linéaire ou ramifié en $C_8$-$C_{22}$, ou des polyoléfines ayant un groupe terminal à insaturation éthylénique, en particulier ayant un groupement terminal (méth)acrylate.

Les premiers types de macromonomères carbonés sont notamment décrits dans les brevets EP 895 467 et EP 96 459 et dans l'article Gillman K.F., Polymer Letters, Vol 5, page 477-481 (1967).

On peut en particulier citer les macromonomères à base de poly(acrylate d'éthyl-2-hexyle) ou de poly(acrylate

de dodécyle) à extrémité mono(méth)acrylate.

Les seconds types de macromonomères sont en particulier décrits dans US 5 625 005 qui mentionne des macromonomères éthylène/butylène et éthylène/propylène à groupement terminal réactif (méth)acrylate.

On peut en particulier citer le méthacrylate de poly(éthylène/butylène), tel que celui commercialisé sous la dénomination Kraton Liquid L-1253 par Kraton Polymers.

Comme macromonomères siliconés, on peut utiliser les monométhacryloxypropyl polydiméthylsiloxanes tels que ceux commercialisés sous la dénomination PS560-K6 par la société United Chemical Technologies Inc. (UCT) ou sous la dénomination MCR-M17 par la société Gelest Inc.

Comme copolymère éthylénique greffé particulièrement préféré dispersé dans une phase grasse liquide non siliconée, on peut utiliser ceux obtenus par polymérisation :

- de l'acrylate de méthyle et du macromonomère polyéthylène/polybutylène à groupement terminal méthacrylate (notamment Kraton L-1253), en particulier dans un solvant choisi parmi l'isododécane, l'isononanoate d'isononyle, l'octyldodécanol, le malate de diisostéaryle, un benzoate d'alkyl $C_{12}$-$C_{15}$ (tel que Finsolv TN) ;
- de l'acrylate de méthoxyéthyle et du macromonomère polyéthylène/polybutylène à groupement terminal méthacrylate (notamment Kraton L-1253), en particulier dans l'isododécane ;
- des monomères acrylate de méthyle / méthacrylate de méthyle et du macromonomère polyéthylène/polybutylène à groupement terminal méthacrylate (notamment Kraton L-1253), en particulier dans l'isododécane ;
- des monomères acrylate de méthyle / acide acrylique et du macromonomère polyéthylène/polybutylène à groupement terminal méthacrylate (notamment Kraton L-1253), en particulier dans l'isododécane ;
- des monomères acrylate de méthyle / méthacrylate de diméthylaminoéthyle et du macromonomère polyéthylène/polybutylène à groupement terminal méthacrylate (notamment Kraton L-1253), en particulier dans l'isododécane ;
- des monomères acrylate de méthyle / méthacrylate de 2-hydroxyéthyle et du macromonomère polyéthylène/polybutylène à groupement terminal méthacrylate (notamment Kraton L-1253), en particulier dans l'isododécane.

Comme polymère acrylique greffé particulièrement préféré dispersé dans une phase grasse liquide siliconée, on peut utiliser ceux obtenus par polymérisation :

- de l'acrylate de méthyle et du macromonomère monométhacryloxypropylpolydiméthylsiloxane ayant un poids moléculaire moyen en poids allant de 800 à 6000 g/mol, en particulier dans le décaméthylcyclopentasiloxane ou le phényltriméthicone ;
- de l'acrylate de méthyle, d'acide acrylique et du macromonomère monométhacryloxypropylpolydiméthylsiloxane ayant un poids moléculaire moyen en poids allant de 800 à 6000 g/mol, en particulier dans le décaméthylcyclopentasiloxane ou le phényltriméthicone.

Selon une autre forme de l'invention, le polymère filmogène peut être choisi parmi les polymères siliconés greffés par des monomères organiques non siliconés. Ces polymères peuvent être liposolubles, lipodispersables, hydrosolubles ou dispersables en milieu aqueux, le cas échéant.

Des exemples de polymères ayant un squelette de polysiloxane greffé par des monomères organiques non siliconés convenables pour une mise en oeuvre de la présente invention, ainsi que leur méthode spécifique de préparation, sont décrits en particulier dans les demandes de brevet EP-A-0 582 152, WO 93/23009 et WO 95/03776.

Selon un mode de réalisation particulier, un polymère filmogène siliconé convenant particulièrement à la mise en oeuvre de la présente invention, peut être un copolymère comportant des groupements carboxylates et des groupements polydiméthylsiloxanes.

Comme copolymères utilisables, on peut citer par exemple les copolymères d'acide acrylique et d'acrylate de stéaryle à greffons polydiméthylsiloxane, les copolymères de méthacrylate de stéaryle à greffons polydiméthylsiloxane, les copolymères d'acide acrylique et de méthacrylate de stéaryle à greffons polydiméthylsiloxane, les copolymères de méthacrylate de méthyle, méthacrylate de butyle, d'acrylate d'éthyl-2-hexyle et de méthacrylate de stéaryle à greffons polydiméthylsiloxane.

Selon un mode de réalisation, une composition selon l'invention peut comprendre en outre au moins un agent auxiliaire de filmification.

## MILIEU PHYSIOLOGIQUEMENT ACCEPTABLE

Une composition selon l'invention comprend un milieu physiologiquement acceptable.

Par "milieu physiologiquement acceptable", on entend désigner un milieu convenant particulièrement à l'application d'une composition selon l'invention sur la peau et/ou les lèvres. Le milieu physiologiquement acceptable est généralement adapté à la nature du support sur lequel doit être appliquée la composition, ainsi qu'à l'aspect sous lequel

la composition est destinée à être conditionnée.

Le milieu physiologiquement acceptable peut comprendre une phase aqueuse et/ou hydrosoluble et/ou une phase grasse.

Selon un mode particulier de réalisation, la phase aqueuse ou la phase grasse peut former la phase continue de la composition.

Cette phase aqueuse peut, le cas échéant, être épaissie, gélifiée ou structurée en y incorporant en outre un gélifiant aqueux traditionnel notamment d'origine minérale comme l'argile par exemple et/ou organique comme un polymère gélifiant aqueux.

Selon un autre mode particulier de réalisation, une composition conforme à l'invention peut se présenter sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de stick, de pâte, de mousse, de dispersion de vésicules notamment de lipides ioniques ou non, de lotion biphase ou multiphase ou de poudre.

Au sens de la présente invention, les émulsions contiennent une phase lipophile, et une phase hydrophile, cette dernière n'étant pas systématiquement de l'eau.

Ainsi, les compositions cosmétiques conformes à l'invention peuvent être sous forme d'émulsion anhydre.

En particulier, la composition peut posséder, par exemple, une phase grasse continue, pouvant contenir moins de 10 % en poids d'eau, notamment moins de 5 % en poids d'eau, voire moins de 1 % en poids d'eau par rapport au poids total de la composition.

En particulier, une composition cosmétique selon l'invention peut être anhydre, c'est-à-dire contenir moins de 5 % en poids, en particulier moins de 3 % en poids, en particulier moins de 2 % en poids, et plus particulièrement moins de 1 % en poids d'eau par rapport au poids total de la composition. Elles peuvent alors se présenter notamment sous forme de gels huileux, de liquides huileux, de pâtes ou de sticks ou encore sous forme de dispersion vésiculaire contenant des liquides ioniques et/ou non ioniques.

PHASE GRASSE

Une composition cosmétique conforme à la présente invention comprend une phase grasse, comprenant au moins une huile volatile telle que décrite ci-dessous.

Elle peut également comprendre une phase grasse notamment choisie parmi une phase grasse liquide, comme des huiles non volatiles, et un corps gras solide à température ambiante (20 - 25 °C) et pression atmosphérique, et leurs mélanges.

On entend par huile, tout corps gras sous forme liquide à température ambiante (20 - 25 °C) et à pression atmosphérique. La phase grasse liquide peut, également, contenir outre des huiles, d'autres composés solubilisés dans les huiles tels que des agents gélifiants et/ou structurants.

La ou les huiles peuvent être présentes à raison de 0,1 à 99 % en poids, en particulier d'au moins 1 à 90 % en poids, plus particulièrement de 5 à 70 % en poids, notamment de 10 à 60 % en poids, voire de 20 à 50 % en poids, par rapport au poids total de la composition cosmétique selon l'invention.

Les huiles volatiles ou non volatiles peuvent être des huiles hydrocarbonées notamment d'origine animale ou végétale, des huiles synthétiques, des huiles siliconées, des huiles fluorées, ou leurs mélanges.

Au sens de la présente invention, on entend par "huile siliconée", une huile comprenant au moins un atome de silicium, et notamment au moins un groupe Si-O.

On entend par "huile hydrocarbonée", une huile contenant principalement des atomes d'hydrogène et de carbone et éventuellement des atomes d'oxygène, d'azote, de soufre et/ou de phosphore.

*Huiles volatiles*

Une composition conforme à l'invention comprend au moins une huile volatile.

Au sens de la présente invention, on entend par "huile volatile", une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa ($10^{-3}$ à 300 mm Hg), en particulier allant de 1,3 Pa à 13 000 Pa (0,01 à 100 mm Hg), et plus particulièrement allant de 1,3 Pa à 1300 Pa (0,01 à 10 mm Hg).

Les huiles hydrocarbonées volatiles peuvent être choisies parmi les huiles hydrocarbonées ayant de 8 à 16 atomes de carbone, et notamment les alcanes ramifiés en $C_8$-$C_{16}$ (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permethyls®.

Comme huiles volatiles, on peut aussi utiliser les silicones volatiles, comme par exemple les huiles de silicones

linéaires ou cycliques volatiles, notamment celles ayant une viscosité ≤ 8 centistokes ($8 \times 10^{-6}$ m2/s), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées telles que le nonafluorométhoxybutane ou le perfluorométhylcyclopentane, et leurs mélanges.

Il est également possible d'utiliser un mélange des huiles précédemment citées.

Une composition cosmétique selon l'invention peut comprendre au moins une huile volatile en une teneur variant d'environ 2 % à environ 70 % en poids, en particulier variant d'environ 5 % à environ 60 % en poids, et plus particulièrement variant d'environ de 10 % à environ 50 % en poids d'huile par rapport au poids total de la composition.

Introduite en quantité suffisante, et en combinaison avec un ester d'acide d'imerdilinoléique et de polyol(s) ou un de ses esters, et un agent filmogène tels que définis précédemment, l'huile volatile présente notamment l'avantage de pouvoir conférer une amélioration de la tenue de la brillance et/ou de la couleur aux compositions cosmétiques selon l'invention.

_Huiles non volatiles_

Au sens de la présente invention, on entend par "huile non-volatile", une huile ayant une pression de vapeur inférieure à 0,13 Pa et notamment des huiles de masse molaire élevée.

Une composition cosmétique selon la présente invention peut comprendre en outre au moins une huile non volatile.

Les huiles non volatiles peuvent notamment être choisies parmi les huiles hydrocarbonées le cas échéant fluorées et/ou les huiles siliconées non volatiles.

Comme huile hydrocarbonée non volatile convenant à la mise en oeuvre de l'invention, on peut notamment citer :

- les huiles hydrocarbonées d'origine animale,
- les huiles hydrocarbonées d'origine végétale telles que les esters de phytostéaryle, tels que l'oléate de phytostéaryle, l'isostéarate de physostéaryle et le glutanate de lauroyl/octyldodécyle/phytostéaryle, par exemple vendu sous la dénomination ELDEW PS203 par AJINOMOTO, les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment des triglycérides héptanoïques ou octanoïques, les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STÉARINERIES DUBOIS ou ceux vendus sous les dénominations MIGLYOL 810®, 812® et 818® par la société DYNAMIT NOBEL,
- les huiles hydrocarbonées d'origine minérale ou synthétique comme par exemple :
    - les éthers de synthèse ayant de 10 à 40 atomes de carbone ;
    - les hydrocarbures linéaires ou ramifiés, d'origine minérale ou synthétique tels que la vaseline, les polydécènes, le polyisobutène hydrogéné tel que le parléam, le squalane et leurs mélanges, et en particulier le polyisobutène hydrogéné,
    - les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit ≥ 10.
    Les esters peuvent être notamment choisis parmi les esters, notamment d'acide gras comme par exemple :
        - ❖ l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol et leurs mélanges, les benzoates d'alcools en $C_{12}$ à $C_{15}$, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle ;

❖ les esters de polyols, et les esters de pentaétrythritol, comme le tétrahydroxystéarate/tétraisostéarate de dipentaérythritol,

❖ les esters de dimères diols et dimères diacides tels que les Lusplan DD-DA5® et Lusplan DD-DA7®, commercialisés par la société NIPPON FINE CHEMICAL et décrits dans la demande FR 03 02809,

- les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges, et
- les carbonates de di-alkyle, les 2 chaînes alkyles pouvant être identiques ou différentes, tel que le dicaprylyl carbonate commercialisé sous la dénomination Cetiol CC®, par Cognis,
- les huiles de silicone non volatiles, comme par exemple les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 Cst, et leurs mélanges,

- et leurs mélanges.

Les huiles non volatiles peuvent être présentes dans une composition selon l'invention en une teneur allant de 5 à 90 % en poids, notamment de 25 % à 80 % en poids, et en particulier de 40 % à 70 % en poids, par rapport au poids total de la composition.

*Corps gras solides*

Une composition selon l'invention peut également comprendre au moins une phase grasse solide choisie parmi les cires, les corps gras pâteux, et leurs mélanges.

La cire est solide à température ambiante (25 °C), à changement d'état solide/liquide réversible, ayant une température de fusion supérieure à 30 °C pouvant aller jusqu'à 200 °C, une dureté supérieure à 0,5 MPa et présentant à l'état solide une organisation cristalline anisotrope.

Elle peut être hydrocarbonée, fluorée et/ou siliconée et être d'origine animale, végétale, minérale ou synthétique.

Elle peut être choisie par exemple parmi la cire d'abeille, la cire de Carnauba, la cire de Candelilla, les cires de paraffine, l'huile de ricin hydrogénée, les cires synthétiques comme les cires de polyéthylène (de préférence de poids moléculaire compris entre 400 et 600) ou de Fischer-Tropsch, les cires de silicone comme les alkyl- ou alkoxy-diméthicone ayant de 16 à 45 atomes de carbone, les cérésines ou les ozokérites, comme par exemple les isoparaffines dont le point de fusion est inférieur à 40 °C, tel que l'EMW-0003, commercialisé par la société NIPPON SEIROU, les oligomères d'$\alpha$-oléfine, tel que les polymères PERFORMA V® 825, 103 et 260, commercialisés par la société NEW PHASE TECHNOLOGIES ; les copolymères éthylène-propylène, tel que le PERFORMALENE® EP 700, et les cires microcristallines dont le point de fusion est supérieur à 85 °C, tel que les HI-MIC® 1070, 1080, 1090 et 3080, commercialisées par NIPPON SEIROU, et leurs mélanges.

Selon un mode particulier de mise en oeuvre, la ou les cires utilisées dans les compositions cosmétique conformes à la présente invention peut ou peuvent être présentes en une teneur variant d'environ 5 à environ 30 %, en particulier d'environ 5 à environ 25 %, en particulier d'environ 10 à environ 20 %, et plus particulièrement d'environ 10 à environ 15 % en poids par rapport au poids total de la composition.

Une composition cosmétique conforme à la présente invention peut également comprendre au moins un composé pâteux.

Par "pâteux" au sens de la présente invention, on entend un composé gras à changement d'état solide/liquide réversible et comportant à la température de 23 °C une fraction liquide et une fraction solide. On entend également par pâteux, le polylaurate de vinyle.

Un composé pâteux au sens de l'invention peut présenter avantageusement une dureté à 20 °C allant de 0,001 à 0,5 MPa, de préférence de 0,002 à 0,4 MPa.

Parmi les composés pâteux susceptibles d'être utilisés dans la composition selon l'invention, on peut citer les

lanolines et les dérivés de lanoline comme les lanolines acétylées, les lanolines oxypropylénées ou le lanolate d'isopropyle, et leurs mélanges. On peut également utiliser des esters d'acides ou d'alcools gras, notamment ceux ayant 20 à 65 atomes de carbone comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut utiliser les dérivés d'huile de ricin hydrogénée, tels que le "THIXINR®" de Rheox.

On peut également citer les polyesters résultant de l'estérification d'un acide carboxylique et d'un ester acide hydroxycarboxylique aliphatique. Par exemple, le RISOCAST® DA-L (ester issu de la réaction d'estérification de l'huile de ricin hydrogéné avec de l'acide dilinoléïque dans des proportions de 2 pour 1) et le RISOCAST® DA-H (ester résultant de l'estérification de l'huile de ricin hydrogénée avec de l'acide isostéarique dans des proportions de 4 pour 3) commercialisés par la société japonaise KOKYU ALCOHOL KOGYO.

Comme composé pâteux convenant avantageusement à la formulation des compositions cosmétiques conformes à la présente invention, on peut faire mention des coco-glycérides hydrogénés.

On peut aussi citer les composés pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55 °C, comme les stéaryl diméthicones notamment ceux vendus par la société DOW CORNING sous les noms commerciaux de DC2503® et DC25514® et leurs mélanges.

## BRILLANCE ET TENUE DE LA BRILLANCE

Par "brillance", on entend désigner la brillance d'une composition telle qu'elle peut être mesurée au moyen d'un brillancemètre de manière conventionnelle, par des méthodes connues de l'homme de l'art.

Par exemple, la brillance peut être mesurée au moyen d'un test réalisé *in vitro* à l'aide d'un brillancemètre MUTI-GLOSS 268 (MINOLTA, Tokyo, Japon).

Un tel test peut être effectué sur un échantillon de peau synthétique Bio-Skin (de type Fat-Skin BEAULAX, Tokyo, Japon) de taille 3 cm x 4,5 cm préparé par application d'une première couche de composition cosmétique, par exemple un rouge à lèvres, dans le sens de la longueur du support, sous forme de bandes contiguës, de manière à couvrir une surface faisant 2,5 cm x 4 cm. Une seconde couche de composition est appliquée sur la première couche de manière similaire à la première avec un décalage dans le sens de la largeur d'environ la moitié de la largeur d'un ruban afin d'obtenir une surface uniformément recouverte.

Après application des deux couches de composition, la brillance est mesurée par un brillancemètre par mesure de la réflectance à un angle d'environ 60° pris par rapport à la perpendiculaire de la surface. La brillance est mesurée avantageusement environ 10 minutes après l'application de la composition cosmétique.

La mesure est répétée, par exemple au moins cinq fois, et une moyenne est réalisée sur les valeurs restantes après élimination de la valeur la plus basse et de la valeur la plus élevée.

Par "tenue de la brillance", on entend désigner la tenue de la brillance d'une composition telle qu'elle peut être mesurée au moyen d'un brillancemètre par des méthodes connues de l'homme de l'art.

Par exemple le protocole tel que défini ci-dessus peut être suivi de l'application d'un tissu sur l'échantillon de peau synthétique comprenant les deux couches de compositions cosmétiques. Un tel tissu peut être par exemple un Kleenex.

Le tissu peut être appliquée au moyen d'une presse par exemple de masse de 1,5 kg, pendant un temps défini, par exemple 2 secondes, de façon contrôlée par un rhéomètre (texture Exponet 32).

Puis, la brillance de l'échantillon est mesurée comme défini ci-dessus, et la valeur obtenue est indicative de la tenue.

Une composition selon l'invention peut présenter une tenue de la brillance supérieure ou égale à environ 4, par exemple supérieur ou égale à environ 5, par exemple supérieur à environ 6.

## TENUE DE LA COULEUR DE LA COMPOSITION

Par "tenue de la couleur", on entend désigner la propriété d'une composition cosmétique selon l'invention à transférer dans une moindre mesure sur des objets avec lesquelles elle peut entrer en contact, et la propriété à résister à l'interaction avec des liquides, comme les larmes, la sueur, ou le contact avec des aliments lors d'un repas, notamment dans le cas d'un rouge à lèvres par exemple, et la propriété à ne pas migrer lors du tracé initial du maquillage, en particulier dans le cas des rouges à lèvres, dans les rides et ridules du contour des lèvres.

Un film de composition cosmétique appliqué sur la peau, les muqueuses et/ou les lèvres peut généralement être altéré lors du contact avec des liquides, notamment de l'eau ou des boissons consommées par exemple lors d'un repas, ou bien encore des huiles comme les huiles alimentaires ou bien encore le sébum ou bien encore de la salive.

Ainsi, la tenue de la couleur d'une composition cosmétique peut être caractérisée par au moins l'un des paramètres suivants : le non transfert, la tenue de la couleur à l'eau, et la tenue de la couleur à l'huile.

La mesure de la tenue de la couleur à l'huile peut être un paramètre adéquat pour caractériser une composition selon l'invention et peut se faire comme suit.

Les mesures sont réalisées, par exemple, sur la face interne de l'avant-bras, lavée et laissée séchée naturellement à température ambiante pendant 5 minutes. La composition cosmétique à tester, par exemple un rouge à lèvres, est appliquée sur cinq zones de la face interne de l'avant-bras. La surface de peau sur laquelle les mesures sont réalisées doit être au moins supérieure 1 $cm^2$. De manière générale, les mesures se font sur des zones circulaires de diamètre environ égal à 3 cm.

Il est nécessaire qu'environ la même quantité de composition cosmétique soit appliquée sur chacune des trois zones. Cela peut être vérifié en mesurant le poids de la composition cosmétique, par exemple le rouge à lèvres, après chacune des applications ou en préparant à l'avance des quantités équivalentes d'échantillon à tester. De manière générale, pour une surface de 1 $cm^2$, une quantité égale à environ 2 mg est nécessaire (si la surface possède un diamètre de 3 cm, alors une quantité d'environ 14 mg est requise).

Après application de la composition cosmétique, la couleur, $L_1*a_1*b_1*(C_I)$, est mesurée en chacune des zones, et la valeur moyenne obtenue correspond à la couleur initiale de la composition. La mesure de la couleur peut être effectuée avec un colorimètre MINOLTA de la série CR300 ou CM500 ou CM1000 ou CM2000. On utilise en particulier le colorimètre MINOLTA de la série CR300.

Le test de tenue de la couleur à l'huile est conduit par application sur les zones à tester d'environ 14 $mg/cm^2$ d'huile de type alimentaire sur chaque zone de l'avant-bras (huile de colza, huile de soja ou huile de tournesol) suivi d'un massage manuel pendant quelques secondes, en particulier pendant 2 à 5 secondes, et plus particulièrement pendant 2 secondes. Une épaisseur d'un mouchoir en papier blanc du commerce tel qu'un mouchoir Kleenex est ensuite appliquée sur la zone pendant environ 5 secondes et à une force d'environ 100 g/f, laquelle force peut être appliquée avec un dynamomètre de pression digital DPZ-5N du constructeur, IMADA Co.Ltd.

La valeur de tenue de la couleur à l'huile, H, est égale à la différence entre la couleur moyenne $L_2*a_2*b_2*(C_P)$ de la composition restant sur l'avant bras après le massage à l'huile et l'application du mouchoir, et la couleur moyenne $L_1*a_1*b_1*(C_I)$ mesurée initialement,

$$\Delta E\,(H) = \sqrt{(L_2^* - L_1^*)^2 + (a_2^* - a_1^*)^2 + (b_2^* - b_1^*)^2}$$

Le test de tenue de la couleur à l'huile est un test permettant notamment d'évaluer le maintien d'une composition cosmétique telle qu'un rouge à lèvres lors d'un repas.

En particulier, la tenue de la couleur à l'huile d'un dépôt d'une composition selon l'invention, une fois étalée sur un support, peut être inférieure ou égale à environ 20, en particulier inférieure ou égale à environ 15, en particulier inférieure ou égale à environ 10, inférieure ou égale à environ 8.

## MATIERE COLORANTE

Une composition cosmétique conforme à l'invention peut, en outre, comprendre au moins une matière colorante.

Une telle matière colorante peut être par exemple choisie parmi les matières colorantes hydrosolubles ou non, liposolubles ou non, organiques ou inorganiques, notamment de type pigments ou nacres, classiquement utilisée dans les compositions cosmétiques.

Par pigments, il faut comprendre des particules blanches ou colorées, minérales ou organiques, insolubles dans une solution aqueuse, destinées à colorer et/ou opacifier le film résultant.

Les pigments peuvent être présents à raison de 0,5 à 30 % en poids, notamment de 5 à 25 % en poids, et en particulier de 10 à 20 % en poids, par rapport au poids total de la composition cosmétique.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères

de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Par "nacres", il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica titane recouvert d'oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA, FLAMENCO et DUOCHROME (sur base de mica) commercialisées par la société ENGELHARD, les nacres TIMIRON commercialisées par la société MERCK, les nacres sur base de mica PRESTIGE commercialisées par la société ECKART et les nacres sur base de mica synthétique SUNSHINE commercialisées par la société SUN CHEMICAL.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté par exemple commercialisées par la société MERCK sous la dénomination Xirona Silver et les nacres orangées rosées vert doré notamment commercialisées par la société MERCK sous la dénomination Indian summer (Xirona) et leurs mélanges.

La composition cosmétique selon l'invention peut comprendre également au moins une matière colorante hydrosoluble ou liposoluble en une teneur allant de 0,5 à 30 % en poids, notamment allant de 5 à 25 % en poids par rapport au poids total de la composition cosmétique.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine Les colorants hydrosolubles sont par exemple le jus de betterave, le bleu de méthylène.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques comme par exemple les pigments monochromatiques.

Au sens de l'invention, "stabilisé" signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :

- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.
Par "dérivés métalliques", on désigne des composés dérivés de métaux notamment des oxydes, des fluorures, des chlorures et des sulfures
A titre illustratif de ces particules, on peut citer des particules d'aluminium, telles que celles commercialisées sous les dénominations STARBRITE 1200 EAC® par la société SIBERLINE et METALURE® par la société ECKART.
On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE 700 de la société ECKART, les particules d'aluminium enrobé de silice commercialisées sous la dénomination VISIONAIRE BRIGHT SILVER de la société ECKART et les particules d'alliage métallique comme des poudres de bronze (alliage cuivre et zinc) enrobé de silice commercialisées sous la dénomination de Visionaire Bright Natural Gold de la société Eckart.
Il peut encore s'agir de particules comportant un substrat de verre comme celles commercialisées par la société NIPPON SHEET GLASS sous les dénominations MICROGLASS METASHINE.
L'agent de coloration goniochromatique peut être choisi par exemple parmi les structures multicouches interférentielles et les agents de coloration à cristaux liquides.
Des exemples de structures multicouche interférentielles symétriques utilisables dans des compositions réalisées conformément à l'invention sont par exemple les structures suivantes : $Al/SiO_2/Al/SiO_2/Al$, des pigments ayant cette structure étant commercialisés par la société DUPONT DE NEMOURS ; $Cr/MgF_2/Al/MgF_2/Cr$, des pigments ayant cette structure étant commercialisés sous la dénomination CHROMAFLAIR par la société FLEX ; $MoS_2/SiO_2/Al/SiO_2/MoS_2$ ; $Fe_2O_3/SiO_2/Al/SiO_2/Fe_2O_3$, et $Fe_2O_3/SiO_2/Fe_2O_3/SiO_2/Fe_2O_3$, des pigments ayant ces structures étant commercialisés sous la dénomination SICOPEARL par la société BASF ; $MoS_2/SiO_2$/mica-oxyde/$SiO_2/MoS_2$ ; $Fe_2O_3/SiO_2$/mica-oxyde/$SiO_2/Fe_2O_3$ ; $TiO_2/SiO_2/TiO_2$ et $TiO_2/Al_2O_3/TiO_2$ ; $SnO/TiO_2/SiO_2/TiO_2/SnO$ ; $Fe_2O_3/SiO_2/Fe_2O_3$ ; SnO/mica/$TiO_2/SiO_2/TiO_2$/mica/SnO, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure $Fe_2O_3/SiO_2/Al/ SiO_2/Fe_2O_3$ on passe du doré-vert au gris-rouge pour des couches de $SiO_2$ de 320 à 350 nm ; du rouge au doré pour des couches de $SiO_2$ de 380 à 400 nm ; du violet au vert pour des couches de $SiO_2$ de 410 à 420 nm ; du cuivre au rouge pour des couches de $SiO_2$ de 430 à 440 nm.
On peut citer, à titre d'exemple de pigments à structure multicouche polymérique, ceux commercialisés par la société 3M sous la dénomination COLOR GLITTER.
Comme particules goniochromatiques à cristaux liquides, on peut utiliser par exemple celles vendues par la société CHENIX ainsi que celle commercialisées sous la dénomination HELICONE® HC par la société WACKER.
Selon un mode de réalisation, une composition selon l'invention peut comprendre au moins une matière colorante par exemple choisie parmi les matières colorantes organiques et les matières colorantes inorganiques, telles que les pigments et les nacres, les matériaux à effet optique spécifique et leurs mélanges.
Selon un mode de réalisation, une composition selon l'invention peut ne pas comprendre plus de 30 % en poids de matière colorante par rapport au poids total de la composition.

## CHARGES

Les compositions cosmétiques conformes à l'invention peuvent également comprendre au moins une charge, de nature organique ou minérale, permettant notamment de leur conférer une stabilité améliorée au regard de l'exsudation.
Par "charge", il faut comprendre les particules incolores ou blanches, solides de toutes formes, qui se présentent sous une forme insoluble et dispersée dans le milieu de la composition. De nature minérale ou organique, elles permettent de conférer du corps ou de la rigidité à la composition, et/ou de la douceur, de la matité et de l'uniformité au maquillage.

Les charges utilisées dans les compositions selon la présente invention peuvent être de formes lamellaires, globulaires, sphériques, de fibres ou de toute autre forme intermédiaire entre ces formes définies.

Les charges selon l'invention peuvent être ou non enrobées superficiellement, et en particulier elles peuvent être traitées en surface par des silicones, des acides aminés, des dérivés fluorés ou toute autre substance favorisant la dispersion et la compatibilité de la charge dans la composition.

Au sens de la présente invention, les termes "charges minérales" et "charges inorganiques" sont utilisés de manière interchangeable.

Parmi les charges minérales utilisables dans les compositions selon l'invention, on peut citer le talc, le mica, la silice, le siloxysilicate de triméthyle, le kaolin, la bentone, le carbonate de calcium précipité, le carbonate et l'hydrogéno-carbonate de magnésium, l'hydroxyapatite, le nitrure de bore, les microsphères de silice creuses (Silice Beads de Maprecos), les microcapsules de verre ou de céramique, les charges à base de silice comme l'Aerosil 200, l'Aerosil 300 ; le Sunsphare L-31, le Sunphare H-31 commercialisés par Asahi Glass ; le Chemicelen commercialisé par Asahi Chemical ; les composites de silice et de dioxyde de titane comme la série TSG commercialisée par Nippon Sheet Glass, et leurs mélanges.

Parmi les charges organiques utilisables dans les compositions selon l'invention on peut citer les poudres de polyamide (Nylon® Orgasol de chez Atochem), de poly-b-alanine et polyéthylène, les poudres de polytétrafluoroéthylène (Téflon®), la lauroyl-lysine, l'amidon, les poudres de polymères de tétrafluoroéthylène, les microsphères creuses de polymères telles l'EXPANCEL (NOBEL INDUSTRIE), le carbonate de calcium précipité, le carbonate et l'hydrogéno-carbonate de magnésium, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, le Polypore® L 200 (Chemdal Corporation), les microbilles de résine de silicone (Tospearl® de Toshiba, par exemple), les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400® ou PLASTIC POWDER D-800® de la société TOSHIKI, et leurs mélanges.

Une charge peut être présente dans une composition cosmétique conforme à l'invention à raison de 0,5 à 40 % en poids de charge par rapport au poids total de la composition, de préférence de 5 à 30 %.

Une charge convenant à l'invention peut être par exemple une charge dont la granulométrie moyenne est inférieure à 100 $\mu$m, notamment comprise entre 1 et 50 $\mu$m, par exemple entre 4 et 20 $\mu$m.

**ADDITIFS**

Les compositions cosmétiques selon l'invention peuvent également comprendre en outre tout additif usuellement utilisé dans le domaine concerné, choisi parmi des agents tensioactifs, des agents gélifiants, des polymères semi-cristallins, des gommes, des agent antioxydants, des huiles essentielles, des conservateurs, des parfums, des neutralisants, des agents hydratants, des agents antiseptiques, des vitamines telles que les vitamines B3 ou E et leurs dérivés, des agents protecteurs contre les UV, et leurs mélanges.

Selon un mode de réalisation, la présente invention a pour objet l'utilisation d'au moins un ester d'acide dimerdilinoléique et de polyol(s) ou d'un de ses esters, dont la viscosité, mesurée à environ 25 °C, est supérieure ou égale à environ 20 000 mPa.s, en association avec au moins une huile volatile et au moins un agent filmogène pour la préparation d'une composition cosmétique de soin et/ou de maquillage de la peau et/ou des lèvres présentant une tenue de la brillance améliorée, voire une tenue de la couleur améliorée.

Notamment, l'ester, l'agent filmogène et l'huile volatile peuvent être, par exemple, tels que définis précédemment.

Une composition conforme à l'invention peut être préparée de manière usuelle par l'homme du métier. Elle peut, ainsi, se présenter sous forme coulée, et par exemple sous la forme d'un stick ou bâton, sous forme de pâte souple dans une bouillotte ou sous la forme de coupelles utilisables par contact direct ou à l'éponge.

Par exemple, elle peut constituer un fond de teint coulé, un fard à joues ou à paupières coulé, notamment coloré, un rouge à lèvres, un brillant pour les lèvres, un produit anti-cernes.

Une composition selon l'invention peut être obtenue par chauffage des différents constituants à la température de fusion des cires les plus élevées, puis par coulage du mélange fondu dans un moule (coupelle ou doigt de gant). Elle peut également être obtenue par extrusion, comme décrit dans la demande EP A 0 667 146.

La présente invention concerne également un procédé de maquillage et/ou de soin de la peau et/ou des lèvres comprenant au moins une étape consistant à appliquer sur au moins une partie d'un support une composition conforme à l'invention.

Selon un autre de ses aspects, la présente invention a pour objet un support synthétique comprenant sur au moins une partie de sa surface une composition conforme à l'invention.

Une composition cosmétique selon l'invention peut notamment se présenter sous la forme d'une composition de maquillage et/ou de soin des lèvres, en particulier un rouge à lèvres, un baume à lèvres ou un gloss.
Au sens de la présente invention, et sans indication contraire, "un" doit s'entendre comme signifiant "au moins un".
Les exemples de compositions ci-après sont donnés à titre illustratif et sans caractère limitatif de l'invention.

**EXEMPLE**

*Rouge à lèvres :*

| | % massique |
|---|---|
| copolymère hexadécène/PVP | 1,82 |
| tri-isostéarate de polyglycéryle-2 | 1,00 |
| copolymère d'isostéarate de polyglycéryle-2 /dimerdilinoléate (HAILUCENT ISDA (HAI)) | 4,00 |
| dimerdilinoléyle dimerdilinoléate (LUSPLAN DD-DA7 (NFC)) | 2,00 |
| bis-béhényl/isostéaryle/phytostéryle dimerdilinoléyle dimerdilinoléate (Plandool-G (NFC)) | 1,00 |
| Polyisobutène hydrogené | 11,00 |
| isononanoate d'isononyle | 2,70 |
| dicaprate de néopentyl glycol | 2,27 |
| phényle triméthicone | 2,70 |
| isododécane | 1,82 |
| D5 | 36,32 |
| PSPA | 3,64 |
| résine MQ | 5,46 |
| BHT | 0,05 |
| polyéthylène (M : 500) | 13,64 |
| cire de Candelilla | 1,82 |
| dioxyde de titane | 0,18 |
| D&C RED 7 W 012 C | 0,41 |
| oxydes de fer | 0,86 |
| YELLOW 5 LAKE | 0,77 |
| BLUE 1 LAKE | 0,18 |
| copolymère di-méthacrylate d'éthylène glycol/méthacrylate de lauryle | 0,91 |
| N-lauroyl L-lysine | 0,91 |
| silice | 0,45 |
| Oxyde de fer recouvert de mica | 2,55 |
| dioxyde de titane recouvert de borosilicate de calcium et d'aluminium | 0,45 |
| dioxyde de titane recouvert de mica | 0,91 |
| Siméthicone | 0,18 |
| Total | 100 |

Mode opératoire

Une phase huileuse est préparée en mélangeant à chaud (environ 95 °C) une partie des huiles, l'agent filmogène, ainsi que le copolymère d'isostéarate de polyglycéryl-2 dimerdilinoléate (HAILUSCENT ISDA).
La phase huileuse ainsi préparée est maintenue sous agitation à environ 95 °C et les charges sont ajoutées au mélange.
Puis sont rajoutés au mélange les cires, les pigments sous la forme d'une pâte pigmentaire, préparée par mélange des matières colorantes avec le triisostéarate de polyglycéryle-2, les huiles restantes, la siméthicone, et le bisbéhényl/isostéaryle/phytostéryle dimerdilinoléyle dimerdilinoléate (Plandool-G).
Le mélange ainsi obtenu est ensuite coulé dans un moule de rouge à lèvres et laissé refroidir jusqu'à l'obtention d'une composition solide.
La tenue de la brillance et la tenue de la couleur de la composition sont mesurées comme décrit précédemment.
La tenue de la brillance est de 7,1 et la tenue de la couleur est de 5,4.

## Revendications

1. Composition cosmétique anhydre de soin et/ou de maquillage de la peau et/ou des lèvres comprenant au moins un ester d'acide dimerdilinoléique et de polyol(s) ou un de ses esters, dont la viscosité, mesurée à environ 25 °C, est supérieure ou égale à environ 20 000 mPa.s, au moins un agent filmogène et au moins une huile volatile.

2. Composition selon la revendication précédente, dans laquelle ledit ester présente un poids moléculaire variant d'environ 2000 à environ 25 000 g/mol, en particulier d'environ 4000 à environ 20 000 g/mol, en particulier d'environ 7000 à environ 15 000 g/mol, et plus particulièrement d'environ 8000 à environ 10 000 g/mol.

3. Composition selon l'une quelconque des revendications précédentes, dans laquelle le polyol est un diol.

4. Composition selon la revendication précédente, dans laquelle l'ester comprend un enchaînement alterné de résidu (s) dimerdilinoléate(s) et de résidu(s) apparenté(s) au(x)dit(s) diol(s).

5. Composition selon la revendication 3 ou 4, dans laquelle le diol est choisi parmi un dimère d'alcool gras, un mono- ou poly-glycérol, un mono- ou poly-alkylène en $C_2$-$_4$ glycol, le 1,4 butanediol et le pentaérythritol.

6. Composition selon la revendication 5, dans laquelle le dimère d'alcool gras est le produit de l'hydrogénation du dimère d'un acide gras obtenu par dimérisation d'un acide gras insaturé en $C_8$ à $C_{34}$, notamment en $C_{12}$ à $C_{22}$, en particulier en $C_{16}$ à $C_{20}$ et plus particulièrement en $C_{18}$.

7. Composition selon l'une quelconque des revendications 4 à 6, dans laquelle chacune des deux extrémités dudit enchaînement porte respectivement un motif OR' et OR", avec R' et R" représentant, indépendamment l'un de l'autre, un atome d'hydrogène ou OR' et OR" représentant, indépendamment l'un de l'autre, un résidu d'un monoalcool hydrocarboné en $C_2$ à $C_{36}$.

8. Composition selon la revendication précédente, dans laquelle R' et R" représentent tous les deux un atome d'hydrogène.

9. Composition selon la revendication 7, dans laquelle OR' et OR" représentent tous les deux un résidu d'un monoalcool hydrocarboné, identique ou différent.

10. Composition cosmétique de soin et/ou de maquillage de la peau et/ou des lèvres comprenant au moins un ester de formule générale (I) suivante :

$$R_3\text{-OCO-}R_1(\text{-COO-}R_2\text{-OCO-}R_1)_n\text{-COO-}R_3 \qquad (I)$$

dans laquelle :

- $COR_1CO$ représente un résidu dimerdilinoléate,
- $OR_2O$ représente un résidu de dimère d'alcool gras,
- $OR_3$ représente un résidu de monoalcool hydrocarboné, et

- n est un entier variant de 1 à 15,

au moins un agent filmogène et au moins une huile volatile.

**11.** Composition selon l'une quelconque des revendications 1 à 7 et 9, comprenant un ester tel que défini selon la revendication 10.

**12.** Composition selon la revendication 10 ou 11, dans laquelle $OR_2O$ représente un résidu dimerdilinoléyle.

**13.** Composition selon l'une quelconque des revendications 10 à 12, dans laquelle $OR_3$ représente un résidu de monoalcool hydrocarboné choisi parmi les résidus béhényle, isostéaryle, phytostéryle et leurs mélanges.

**14.** Composition cosmétique de soin et/ou de maquillage de la peau et/ou des lèvres comprenant au moins un ester de formule générale (II) suivante :

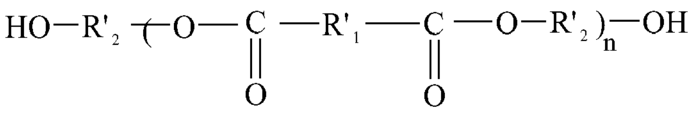

(II)

dans laquelle :

- n est un entier variant de 1 à 15,
- COR'1CO représente un résidu dimerdilinoléate,
- OR'2O représente un résidu diglycéryle de formule générale (III) suivante :

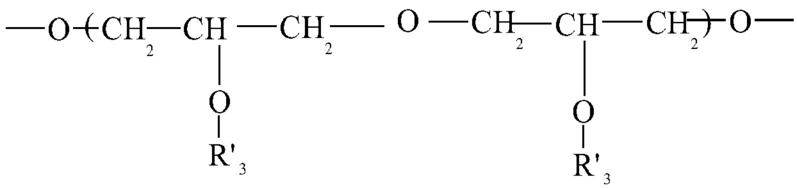

(III)

dans laquelle :

- $R'_3$ représente H ou $OR'_3$ représente un résidu d'acide gras, au moins un agent filmogène et au moins une huile volatile.

**15.** Composition selon l'une quelconque des revendications 1 à 8, comprenant un ester tel que défini selon la revendication 14.

**16.** Composition selon la revendication 14 ou 15, dans laquelle le résidu d'acide gras figuré par $OR'_3$ est un résidu d'isostéaryle.

**17.** Composition selon l'une quelconque des revendications précédentes, dans laquelle ledit ester est choisi parmi les esters de nomenclature INCI suivante : le copolymère d'isostéarate de polyglycéryle-2/dimerdilinoléate, le bis-béhényl/isostéaryl/phytostéryl dimerdilinoléyle dimerdilinoléate, et leurs mélanges.

**18.** Composition selon l'une quelconque des revendications précédentes, **caractérisée en ce qu'**elle comprend de 0,1 % à 80 %, en particulier de 2 % à 70 %, et plus particulièrement de 30 % à 50 % en poids dudit ester par rapport au poids total de la composition.

**19.** Composition selon l'une quelconque des revendications précédentes, dans laquelle l'agent filmogène est un polymère filmogène.

**20.** Composition selon la revendication précédente, dans laquelle le polymère filmogène est choisi parmi les polymères

filmogènes solubles dans un milieu liquide organique, les polymères filmogènes dispersibles dans un milieu liquide organique, les polymères filmogènes sous forme de dispersions aqueuses de particules de polymères, les polymères filmogènes hydrosolubles, et leurs mélanges.

**21.** Composition selon l'une quelconque des revendications précédentes, dans laquelle l'agent filmogène est présent en une teneur variant d'environ 0,1 à environ 60 % en poids, en particulier variant d'environ 1 à environ 50 % en poids, en particulier variant d'environ 1 à environ 40 % en poids, en particulier variant d'environ 2 à environ 30 % en poids, et plus particulièrement variant d'environ 5 à environ 25 % en poids, par rapport au poids total de la composition.

**22.** Composition selon l'une quelconque des revendications précédentes, comprenant en outre au moins un agent auxiliaire de filmification.

**23.** Composition selon l'une quelconque des revendications précédentes, dans laquelle l'huile volatile est choisie parmi :

- les huiles hydrocarbonées ayant de 8 à 16 atomes de carbone, et notamment les alcanes ramifiés en $C_8$-$C_{16}$ comme l'isododécane, l'isodécane, l'isohexadécane,
- les huiles de silicones linéaires ou cycliques, notamment celles ayant une viscosité ≤ 8 centistokes (8 $10^{-6}$ $m^2/s$), et en particulier l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane,
- les huiles fluorées telles que le nonafluorométhoxybutane ou le perfluorométhylcyclopentane, et
- leurs mélanges.

**24.** Composition selon l'une quelconque des revendications précédentes, dans laquelle l'huile volatile est présente en une teneur variant d'environ 2 % à environ 70 %, en particulier d'environ 5 % à environ 60 % en poids, et plus particulièrement d'environ 10 % à environ 50 % en poids d'huile par rapport au poids total de la composition.

**25.** Composition selon l'une quelconque des revendications précédentes, comprenant en outre au moins une huile non volatile choisie parmi des huiles hydrocarbonées d'origine animale, des huiles hydrocarbonées d'origine végétale, des huiles hydrocarbonées d'origine minérale ou synthétique, et leurs mélanges.

**26.** Composition selon l'une quelconque des revendications précédentes, **caractérisée par le fait qu'**elle comprend en outre au moins une phase grasse solide choisie parmi les corps gras pâteux, les cires, et leurs mélanges.

**27.** Composition selon l'une quelconque des revendications précédentes, comprenant en outre au moins une matière colorante.

**28.** Composition selon la revendication précédente, dans laquelle la matière colorante est choisie parmi les matières colorantes organiques et les matières colorantes inorganiques, telles que les pigments et les nacres, les matériaux à effet optique spécifique et leurs mélanges.

**29.** Composition selon l'une quelconque des revendications précédentes, **caractérisée en ce qu'**elle se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de stick, de pâte, de mousse, de dispersion de vésicules notamment de lipides ioniques ou non, de lotion biphase ou multiphase ou de poudre.

**30.** Composition selon l'une quelconque des revendications 10 à 29, **caractérisée en ce qu'**elle est anhydre.

**31.** Composition selon l'une quelconque des revendications précédentes, **caractérisée en ce qu'**il s'agit d'une composition de maquillage et/ou de soin des lèvres.

**32.** Utilisation d'au moins un ester d'acide dimerdilinoléique et de polyol(s) ou d'un de ses esters, dont la viscosité, mesurée à environ 25 °C, est supérieure ou égale à environ 20 000 mPa.s, en association avec au moins une huile volatile et au moins un agent filmogène pour la préparation d'une composition cosmétique de soin et/ou de maquillage de la peau et/ou des lèvres présentant une tenue de la couleur améliorée.

**33.** Utilisation selon la revendication précédente, dans laquelle ledit ester est tel que défini selon l'une quelconque des revendications 1 à 17.

**34.** Utilisation selon la revendication 32 ou 33, dans laquelle l'agent filmogène est tel que défini selon l'une quelconque des revendications 19 à 21.

**35.** Utilisation selon l'une quelconque des revendications 33 à 35, dans laquelle l'huile volatile est telle que définie selon la revendication 23 ou 24.

**36.** Procédé de maquillage et/ou de soin de la peau et/ou des lèvres comprenant au moins une étape consistant à appliquer sur au moins une partie d'un support une composition telle que définie selon l'une quelconque des revendications 1 à 31.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0750848 A
- JP 2004256515 A
- JP 2005179377 A
- FR 0450540
- WO 04028487 A
- US 20030103918 A
- US 20030049216 A
- FR 0302849 W
- FR 0303709 W
- EP 1411069 A
- WO 04055081 A
- EP 895467 A
- EP 96459 A
- US 5625005 A
- EP 0582152 A
- WO 9323009 A
- WO 9503776 A
- FR 0302809
- EP 542669 A
- EP 787730 A
- EP 787731 A
- WO 9608537 A
- EP 0667146 A


### Littérature non-brevet citée dans la description


- **GILLMAN K.F.** *Polymer Letters,* 1967, vol. 5, 477-481